# EUROPEAN PATENT APPLICATION

(11) **EP 3 782 645 A1**
(43) Date of publication of application: **24.02.2021**
(21) Application number: 19788111.3
(22) Date of filing: 11.04.2019
(51) Int. Cl.: A61K 39/395, A61K 31/664, A61K 31/7076, A61P 35/00

(54) **ANTICANCER T CELL THERAPY PRODUCT-ASSISTING COMPOSITION COMPRISING DEPLETING ANTI-CD4 MONOCLONAL ANTIBODY AND USE THEREOF**

(30) Priority: 17.04.2018 KR 20180044411
(71) Applicant: National Cancer Center, Goyang-si, Gyeonggi-do 10408 (KR); Eutilex Co., Ltd., Seoul 08594 (KR)
(72) Inventor: CHOI, Beom Kyu, Paju-si, Gyeonggi-do 10893 (KR); KIM, Seon Hee, Goyang-si, Gyeonggi-do 10406 (KR); KWON, Byoung Se, Goyang-si, Gyeonggi-do 10235 (KR); KIM, Young Ho, Goyang-si, Gyeonggi-do 10344 (KR)
(74) Representative: Dehns
(86) International application number: PCT/KR2019/004349
(87) International publication number: WO 2019/203497

(57) **Abstract**

The present invention relates to an anticancer T cell therapy product-assisting composition comprising a depleting anti-CD4 monoclonal antibody and a use thereof. Accordingly, the composition comprising a depleting anti-CD4 monoclonal antibody according to the present invention is able to maximize the anticancer effect of a cancer antigen-specific anticancer T cell therapy product by maintaining an immunodeficient state and is thus effective. In addition, when administered twice or more times at regular intervals of 5 to 8 days, the composition exhibits a far superior effect.

## Description

### [Technical Field]

This application claims priority to and the benefit of Korean Patent Application No. 10-2018-0044411, filed on April 17, 2018, the disclosure of which is incorporate herein by reference in its entirety.

The present invention relates to a method of preventing or treating cancer, which includes inducing transient immunodeficiency in a cancer patient or inducing continuous immunodeficiency using a depleting anti-CD4 monoclonal antibody, and a method of maintaining immunodeficiency.

In addition, the present invention relates to a composition for maintaining immunodeficiency, which includes a depleting anti-CD4 monoclonal antibody and a composition for helping an anticancer T cell therapy product.

In addition, the present invention relates to a pharmaceutical composition used in prevention or treatment of cancer, which includes a depleting anti-CD4 monoclonal antibody, a cancer antigen-specific CD8 T cell, an immunodeficiency inducer, and IL-2.

### [Background Art]

The concept of treating cancer using T cells was first suggested in the mid-1960s, but at the beginning of the research, there were no practical methods of isolating T cells recognizing cancer cells, and a method of isolating T cells from an animal immunoinjected with a cancer antigen was used. Approximately 20 years after the concept of a T cell therapy product was first suggested, a method of isolating and culturing a tumor-infiltrating lymphocyte (TIL) from cancer tissue of a human was established. Afterward, cancer antigen-specific cytotoxic T lymphocytes (CTLs) were isolated to be used as a T cell therapy product.

Among various cancer antigens, an autologous cancer antigen is a protein which is highly expressed in cancer cells, compared with normal cells, and generally a protein necessary for the growth and survival of cancer cells. Therefore, autologous cancer antigens overexpressed in cancer cells, such as human telomere reverse transcriptase (hTERT), Wilm's tumor antigen 1 (WT-1), NY-ESO-1, and melanoma-associated antigen (MAGE), have been used as targets for developing an anticancer immunotherapy product in various ways. However, compared with cancer antigen-specific T cell therapy products exhibiting a high cure rate, T cells targeting an autologous cancer antigen showed a very low cure rate in cancer patients. For this reason, a method of using polyclonal CD8 T cells including various types of clones recognizing an autologous cancer antigen or using TIL cells including CD4 and CD8 T cells targeting various cancer antigens of cancer is being tried.

To prevent the occurrence of an autoimmune disease in the body, T cells recognizing an autoantigen with high affinity are removed by immune tolerance. Accordingly, since T cells recognizing an autologous cancer antigen present in the body basically have a T cell receptor (TCR) with low affinity, their anticancer effect is also lowered. This means that it is necessary to induce and increase a response of T cells to a cancer antigen to cure recurring cancer having no response to standard treatment.

As a method of overcoming the limitation of autologous cancer antigen-specific T cells with a low anticancer effect, most anticancer T cell therapy products induce transient immunodeficiency by administration of a chemotherapeutic agent, and then maximize an anticancer effect by administering T cells. The transient immunodeficiency (lymphopenia) by administering a chemotherapeutic agent has an effect of promoting the *in vivo* concentration of a T cell growth factor, removal of immunosuppressor cells, and homeostatic proliferation of administered T cells. However, the transient immunodeficiency phenomenon by a chemotherapeutic agent is maintained only for approximately 7 to 14 days, and the anticancer effect of the administered T cells is reduced over time.

In Korean Patent No. 10-1503341, a method of selectively isolating autologous cancer antigen-specific CD8⁺ T cells present at an ultimately low proportion in the body and massively proliferating the T cells is disclosed. However, no research for improving an immunodeficiency phenomenon to enhance the anticancer effect of an anticancer agent has been reported.

Therefore, the inventors studied whether an anticancer effect of administered T cells can increase by extending an immunodeficiency phenomenon applied when a T cell therapy product is used, and confirmed that an anticancer effect of cell therapy using cancer antigen-specific T cells can be maximized by continuously inducing partial lymphodepletion through periodic administration of a depleting anti-CD4 monoclonal antibody. Thus, the present invention was completed.

### [Disclosure]

### [Technical Problem]

Therefore, as a result of earnest attempts to provide a method of enhancing an anticancer effect of an anticancer T cell therapy product by maintaining an immunodeficient state of a cancer patient, the inventors confirmed that, when a depleting anti-CD4 monoclonal antibody is used, an immunodeficient state may be sufficiently maintained, and thus the present invention was completed.

Accordingly, the present invention is directed to providing a method of preventing or treating cancer, which includes:
i) inducing transient immunodeficiency in a cancer patient;
ii) administering cancer antigen-specific CD8 T cells and IL-2; and
iii) inducing continuous immunodeficiency.

The present invention is also directed to providing a method of maintaining immunodeficiency, which includes:
i) inducing transient immunodeficiency in a cancer patient; and
ii) administering a depleting anti-CD4 monoclonal antibody to the cancer patient in which the transient immunodeficiency is induced.

The present invention is also directed to providing a composition for maintaining immunodeficiency or a composition for helping an anticancer T cell therapy product, which includes a depleting anti-CD4 monoclonal antibody.

The present invention is also directed to providing a pharmaceutical composition used in prevention or treatment of cancer, which includes a depleting anti-CD4 monoclonal antibody, cancer antigen-specific CD8 T cells, an immunodeficiency inducer and IL-2.

### [Technical Solution]

To attain the above-described objects, the present invention may provide a method of preventing or treating cancer, which includes:
i) inducing transient immunodeficiency in a cancer patient;
ii) administering cancer antigen-specific CD8 T cells and IL-2; and
iii) inducing continuous immunodeficiency.

According to an exemplary embodiment of the present invention, the transient immunodeficiency may be induced by irradiation or administering of an anticancer agent.

According to an exemplary embodiment of the present invention, the anticancer agent may be one or more selected from cyclophosphamide and fludarabine.

According to an exemplary embodiment of the present invention, the cancer antigen may be any one or more autologous cancer antigens selected from the group consisting of human telomere reverse transcriptase (hTERT), Wilm's tumor antigen 1 (WT-1), NY-ESO-1, melanoma-associated antigen (MAGE), carcinoembryonic antigen (CEA), CA-125, MUC-1 and melanoma antigen recognized by T cells 1 (MART-1).

According to an exemplary embodiment of the present invention, the continuous immunodeficiency may be induced by a depleting anti-CD4 monoclonal antibody.

According to an exemplary embodiment of the present invention, the depleting anti-CD4 monoclonal antibody may be administered twice or more at intervals of approximately 5 to 8 days.

According to an exemplary embodiment of the present invention, the cancer of Step i) may be any one selected from the group consisting of lung cancer, stomach cancer, breast cancer, colon cancer, liver cancer, prostate cancer, uterine cancer, brain cancer and sarcomas.

The present invention may also provide a method of maintaining immunodeficiency, which includes:
i) inducing transient immunodeficiency in a cancer patient; and
ii) administering a depleting anti-CD4 monoclonal antibody to the cancer patient in which the transient immunodeficiency is induced.

According to an exemplary embodiment of the present invention, the cancer of Step i) may be any one selected from the group consisting of lung cancer, stomach cancer, breast cancer, colon cancer, liver cancer, prostate cancer, uterine cancer, brain cancer and sarcomas.

According to an exemplary embodiment of the present invention, the depleting anti-CD4 monoclonal antibody may be administered twice or more at intervals of approximately 5 to 8 days.

The present invention may also provide a composition for maintaining immunodeficiency, which includes a depleting anti-CD4 monoclonal antibody.

According to an exemplary embodiment of the present invention, the composition may be administered twice or more at intervals of approximately 5 to 8 days.

According to an exemplary embodiment of the present invention, in the case of treatment of the composition, a period of maintaining immunodeficiency may be approximately 10 days or more after the treatment of the composition.

The present invention may also provide a composition for helping an anticancer T cell therapy product, which includes a depleting anti-CD4 monoclonal antibody.

According to an exemplary embodiment of the present invention, the composition may be administered twice or more at intervals of approximately 5 to 8 days.

According to an exemplary embodiment of the present invention, the cancer may be any one selected from the group consisting of lung cancer, stomach cancer, breast cancer, colon cancer, liver cancer, prostate cancer, uterine cancer, brain cancer and sarcomas.

The present invention may also provide a pharmaceutical composition used in prevention or treatment of cancer, which includes a depleting anti-CD4 monoclonal antibody, a cancer antigen-specific CD8 T cell, an immunodeficiency inducer, and IL-2.

According to an exemplary embodiment of the present invention, the anticancer agent may be one or more selected from the group consisting of cyclophosphamide and fludarabine.

According to an exemplary embodiment of the present invention, the cancer may be any one selected from the group consisting of lung cancer, stomach cancer, breast cancer, colon cancer, liver cancer, prostate cancer, uterine cancer, brain cancer and sarcomas.

Hereinafter, the terms of the present invention will be described.

The term "depleting anti-CD4 monoclonal antibody" used herein refers to a monoclonal antibody which removes or inhibits lymphocyte CD4 cells.

The term "composition for helping an anticancer T cell therapy product" used herein refers to a composition for improving, enhancing or increasing an anticancer effect by an anticancer T cell therapy product.

The term "partial immunodeficiency" used herein refers to deficiency caused by removing or suppressing some of the various immune cells.

Hereinafter, the present invention will be described in detail.

As described above, in the conventional art, to improve the anticancer effect of an anticancer T cell therapy product, transient immunodeficiency was induced, but due to a short period of the transient immunodeficiency, the effect of a cell therapy product is insignificant. As a method that overcomes this, no research on increasing the anticancer effect of an anticancer agent by improving an immunodeficiency phenomenon has not been reported.

In the case of the composition including the depleting anti-CD4 monoclonal antibody according to the present invention, since the anticancer effect of a cancer antigen-specific anticancer T cell therapy product can be maximized by maintaining an immunodeficient state, it is effective in preventing or treating cancer, or maintaining the immunodeficient state of a cancer patient.

Therefore, the present invention provides a method of preventing or treating cancer, which includes:
i) inducing transient immunodeficiency in a cancer patient;
ii) administering cancer antigen-specific CD8 T cells and IL-2; and
iii) inducing continuous immunodeficiency.

The "transient immunodeficiency" refers to temporal degradation of immunity of a cancer patient through irradiation or administration of an anticancer agent, and may exhibit an immunodeficient state for approximately 7 to 14 days.

The irradiation may be total body irradiation in a range of conventionally usable doses, but the present invention is not limited thereto.

The anticancer agent is preferably included at 100 to 300 mg/kg, and more preferably, 150 to 250 mg/kg. When the anticancer agent is included at more than 300 mg/kg, immunodeficiency or an anticancer effect may increase, but the anticancer agent may be fatal to the body, causing side effects such as death.

The anticancer agent is preferably one or more selected from the group consisting of cyclophosphamide and fludarabine, but any anticancer agent used to induce immunodeficiency before T cell therapy can be used without limitation.

The cancer antigen refers to any cancer antigen, for example, a cancer antigen encoded in an MAGE gene family or generated by gene shift or mutation, a cancer antigen expressed due to the surplus of cancer cells, a carcinogenic virus antigen, a tumor fetal antigen, a prostate-specific antigen, an autologous cancer antigen or a differentiation antigen. However, the cancer antigen is preferably an autologous cancer antigen, and any one or more autologous cancer antigens selected from the group consisting of human telomere reverse transcriptase (hTERT), Wilm's tumor antigen 1 (WT-1), NY-ESO-1, melanoma-associated antigen (MAGE), carcinoembryonic antigen (CEA), CA-125, MUC-1 and melanoma antigen recognized by T cells 1 (MART-1).

The cancer antigen-specific CD8 T cells are preferably administered at a concentration of 1 × 10⁵ cells/500 µL to 1 × 10⁸ cells/500 µL, and more preferably, 1 × 10⁶ cells/500 µL to 1 × 10⁷ cells/500 µL. The IL-2 has an effect of proliferating the CD8 T cells in a large amount, and may be administered at a dose of 5,000 to 50,000 IU, and more preferably 7,000 to 20,000 IU.

The term "continuous immunodeficiency" refers to maintenance of an immunodeficient state for a longer time than transient immunodeficiency, so that the immunodeficiency may be maintained for approximately 10 days or more. More preferably, the period may be approximately 15 days or more and 60 days or less, and most preferably approximately 20 days or more and 45 days or less.

The continuous immunodeficiency of the present invention may be induced by a depleting anti-CD4 monoclonal antibody which can induce partial immunodeficiency by binding to CD4 T cells. The depleting anti-CD4 monoclonal antibody may be administered twice or more at intervals of approximately 5 to 8 days, more preferably, approximately four to eight times at intervals of approximately 6 to 7 days, and most preferably, five to six times. When the depleting anti-CD4 monoclonal antibody is administered once or less, the CD8 T cell proportion in the body may be more rapidly reduced than that before the depleting anti-CD4 monoclonal antibody is administered. When the depleting anti-CD4 monoclonal antibody is administered at intervals of 4 days or less or 8 days or more, the immunodeficiency effect may not be stably maintained, resulting in reduced efficiency.

The cancer may be any one selected from the group consisting of lung cancer, stomach cancer, breast cancer, colon cancer, liver cancer, prostate cancer, uterine cancer, brain cancer and sarcomas, but as long as it corresponds to solid cancer, it may be applied without limitation.

In addition, the present invention provides a method of maintaining immunodeficiency, which includes:
i) inducing transient immunodeficiency in a cancer patient; and
ii) administering a depleting anti-CD4 monoclonal antibody to the cancer patient in which the transient immunodeficiency is induced.

Since the cancer and the transient immunodeficiency have the same meanings as used in the method of preventing or treating cancer, the descriptions will be omitted.

In addition, the present invention also provides a composition for maintaining immunodeficiency or a composition for helping an anticancer T cell therapy product, which includes a depleting anti-CD4 monoclonal antibody.

When the composition for maintaining immunodeficiency is treated, a period of maintaining immunodeficiency may be maintained for approximately 10 days or more, preferably, approximately 15 days or more and 60 days or less, and more preferably, approximately 20 days or more and 45 days or less, after the treatment of the composition.

The composition for maintaining immunodeficiency or composition for helping an anticancer T cell therapy product is preferably administered twice or more at intervals of approximately 5 to 8 days, and more preferably, approximately four to eight times at intervals of approximately 6 to 7 days, and most preferably, five to six times. When the composition is administered once or less, the CD8 T cell proportion in the body may be more rapidly reduced than that before the depleting anti-CD4 monoclonal antibody is administered. The composition is administered at intervals of 4 days or less or 8 days or more, the immunodeficiency effect may not be stably maintained, resulting in reduced efficiency.

Accordingly, as the composition for helping an anticancer T cell therapy product including the depleting anti-CD4 monoclonal antibody of the present invention allows an immunodeficiency phenomenon to be maintained, the anticancer effect of the anticancer T cell therapy product may be maximized, and particularly, when the composition is administered twice or more at intervals of approximately 5 to 8 days, compared with the cancer antigen-specific T cell therapy product, it exhibits a higher anticancer effect.

The present invention also provides a pharmaceutical composition used in prevention or treatment of cancer, which includes a depleting anti-CD4 monoclonal antibody, cancer antigen-specific CD8 T cells, an immunodeficiency inducer and IL-2.

The immunodeficiency inducer is used to induce transient immunodeficiency, and may be one or more selected from the group consisting of cyclophosphamide and fludarabine, but any anticancer agent used to induce immunodeficiency before T cell therapy can be used without limitation. The immunodeficiency inducer is preferably included at 100 to 300 mg/kg, and more preferably, 150 to 250 mg/kg. When the anticancer agent is included at more than 300 mg/kg, immunodeficiency or an anticancer effect may increase, but the anticancer agent may be fatal to the body, causing side effects such as death. The IL-2 may have an effect of proliferating the CD8 T cells in a large amount, and may be contained at a dose of 5,000 to 50,000 IU, and more preferably 7,000 to 20,000 IU.

The depleting anti-CD4 monoclonal antibody of the present invention induces partial immunodeficiency by binding to CD4 T cells. When the depleting anti-CD4 monoclonal antibody is administered once, the partial immunodeficiency may be maintained for approximately 5 to 7 days.

The cancer antigen of the present invention is preferably any one or more autologous cancer antigens selected from the group consisting of human telomere reverse transcriptase (hTERT), Wilm's tumor antigen 1 (WT-1), NY-ESO-1, melanoma-associated antigen (MAGE), carcinoembryonic antigen (CEA), CA-125, MUC-1 and melanoma antigen recognized by T cells 1 (MART-1), but any mutant protein associated with a cancer antigen may be applied without limitation.

The pharmaceutical composition for preventing or treating cancer of the present invention is preferably administered twice or more at intervals of approximately 5 to 8 days, more preferably, approximately four to eight times at intervals of approximately 6 to 7 days, and most preferably, five to six times. When the anticancer agent is administered once or less, the CD8 T cell proportion in the body may be more rapidly reduced than that before the depleting anti-CD4 monoclonal antibody is administered. When the anticancer agent is administered at intervals of 4 days or less or 8 days or more, the immunodeficiency effect may not be stably maintained, resulting in reduced efficiency.

The immunodeficiency inducer of the present invention is preferably an anticancer agent, but the present invention is not limited thereto. It is used to induce transient immunodeficiency, is preferably contained at 100 to 300 mg/kg, and more preferably 150 to 250 mg/kg. When the anticancer agent is contained at more than 300 mg/kg, immunodeficiency or an anticancer effect may increase, but the anticancer agent may be fatal to the body, causing side effects such as death.

The anticancer agent of the present invention is preferably one or more selected from the group consisting of cyclophosphamide and fludarabine, but any anticancer agent used to induce immunodeficiency before T cell therapy can be used without limitation.

The cancer is preferably any one selected from the group consisting of lung cancer, stomach cancer, breast cancer, colon cancer, liver cancer, prostate cancer, uterine cancer, brain cancer and sarcomas, but when the cancer corresponds to solid cancer, it can be applied without limitation.

### [Advantageous Effects]

Therefore, when a depleting anti-CD4 monoclonal antibody of the present invention is used in anticancer therapy, an immunodeficient state of a cancer patient is maintained, which is effective because the anticancer effect of the cancer antigen-specific anticancer T cell therapy product can be maximized. In addition, when the depleting anti-CD4 monoclonal antibody or a composition including the same is administered twice or more at intervals of 5 to 8 days, an effect of maintaining an immunodeficient state is more highly exhibited.

### [Description of Drawings]

FIG. 1A shows that, when T cell- and B cell-deficient mice are treated with Pmel-1-specific CD8 T cells (aPmel-1) and additionally treated with whole body radiotherapy and IL-2 administration, the anticancer effect of aPmel-1 increases, resulting in a decreased size of cancer cells.
FIG. 1B shows mice in each group on days 18 and 90 after the drug treatment.
FIG. 2A shows the slowdown of the growth of cancer cells by an increase in dose of cyclophosphamide (CTX) and additional administration of aPmel-1 and IL-2.
FIG. 2B shows the increase in survival rate of mice by an increase in dose of CTX and additional administration of aPmel-1 and IL-2.
FIG. 2C shows the change in cell number of an inguinal lymph node according to a dose of CTX.
FIG. 2D shows the change in cell number of the spleen according to a dose of CTX.
FIG. 3A shows the process of maintaining an immunodeficient state for increasing the anticancer effect of aPmel-1 using a depleting anti-CD4 monoclonal antibody (dCD4).
FIG. 3B shows that the growth of cancer cells goes more slowly by increasing the anticancer effect of aPmel-1 when a depleting anti-CD4 monoclonal antibody is added during aPmel-1 treatment.
FIG. 3C shows that the survival rates of mice increase when a depleting anti-CD4 monoclonal antibody is added during aPmel-1 treatment.
FIG. 3D shows the change in proportion of CD8 cells administered to mouse CD45-positive cells to which a depleting anti-CD4 monoclonal antibody is additionally treated during aPmel-1 treatment.
FIG. 3E shows that the total cell number and CD8 cell number of a mouse additionally treated with a depleting anti-CD4 monoclonal antibody during aPmel-1 treatment being increased.

### [Modes of the Invention]

### [Example 1]

### Confirmation of anticancer effect of only CD8 T cells

Excluding other immune cells with an anticancer effect, the anticancer effect of only cancer antigen-specific CD8 T cells was intended to be evaluated.

Specifically, 2 × 10⁵ B16-F10 melanoma cancer cells were subcutaneously injected into the dorsal area of a T cell or B cell-deficient RAG2^{-/-} mouse to induce the formation of cancer tissue. At the same time, a cell suspension was prepared by collecting the lymph node and spleen of thymocyte antigen 1.1 (Thy 1.1) + premelanosome protein-1 (Pmel-1) transgenic mice, and then CD8 T cells were isolated using anti-CD8 microbeads (Miltenyi Biotec). The isolated cells were suspended in a 10% fetal bovine serum (FBS)-containing RPMI1640 medium (Welgene) at a concentration of 2 × 10⁶ cells/mL, followed by dispensing into a culture dish. After adding 5 µg/mL of hgp100 peptide (KVPRNQDWL, aa 25-33 of human gp100, Peptron), the cells were incubated for 2 days, thereby preparing activated Pmel-1 CD8 T cells (aPmel-1).

For an aPmel-1-administered group, aPmel-1 was washed with PBS twice, and administered into the RAG2^{-/-} mice at a dose of 2 × 10⁶ cells/500 µL/mouse through intravenous injection at 5 days after B16-F10 implantation. For a transient immunodeficiency-induced group, 6 hours before the administration of the Pmel-1 CD8 T cells, 6Gy irradiation was applied to the entire body to induce total body irradiation (TBI). For an IL-2-administered group, after aPmel-1 administration, 10,000 IU of recombinant human IL-2 was intraperitoneally injected once a day for 3 days.

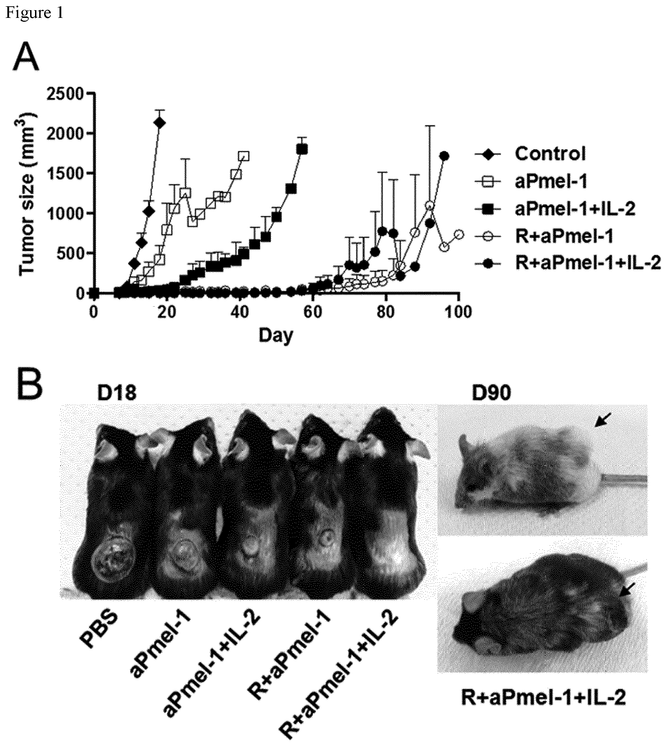

As a result, as shown in FIG. 1A, after only 20 days, the cancer cell sizes in B16-F10 only-implanted mice (Control) exceeded 2000 mm³. In the mice into which only aPmel-1 was intravenously injected, it was shown that the growth of cancer tissue slowed down, and the mice survived for approximately 40 days. When IL-2 was administered in addition to aPmel-1, the cancer tissue grew more slowly, and some mice survived for up to 60 days. When transient immunodeficiency was induced by radiotherapy and then aPmel-1 was administered, some mice survived for up to 100 days, and even when IL-2 was administered additionally, a result similar to the previous result was shown.

In addition, as shown in FIG. 1B, when cancer tissue sizes of mice in each experimental group were analyzed at 18 days after the cancer cell administration, in the experimental group to which aPmel-1 and IL-2 were administered as well as irradiation, almost no cancer tissue was observed. Nevertheless, after approximately 60 days, the cancer tissue began to grow, and at approximately 90 days, the cancer tissue had considerably grown and thus could be visually observed. This shows that, although vitiligo, which is a type of autoimmune disease, was observed as a strong immune response against aPmel-1, B16-F10 cancer cells avoided this and grew again. Therefore, it was determined that B16-F10 cannot be completely removed only with administered aPmel-1.

### [Example 2]

### Confirmation of anticancer effect of CD8 T cells and determination of dose of added transient immunodeficiency inducer

In Example 1, it had been confirmed that cancer cells cannot be completely removed only with aPmel-1, and thus to overcome this, it was expected that the activity of other immune cells such as T cells is needed. Therefore, in a normal immune system, it was intended to determine a dose of an added transient immunodeficiency inducer as well as confirmation of the anticancer effect of cancer antigen-specific CD8 T cells.

Specifically, 2 × 10⁵ B16-F10 melanoma cancer cells were subcutaneously injected into the dorsal area of a C57BL/6 mouse to induce the formation of cancer tissue. At the same time, aPmel-1 was prepared in the same manner as in Example 1. aPmel-1 and IL-2 were administered in the same manner as in Example 1, and transient immunodeficiency was induced by intraperitoneally injecting 150, 200 and 300 mg/kg of cyclophosphamide (CTX) two days before the aPmel-1 administration. In addition, to confirm a period of maintaining transient immunodeficiency according to the dose of CTX, 150, 200 and 300 mg/kg of CTX was intraperitoneally injected into C57BL/6 mice once, and an inguinal lymph node and the spleen of the mice were collected, followed by single cell suspension and then counting a cell number.

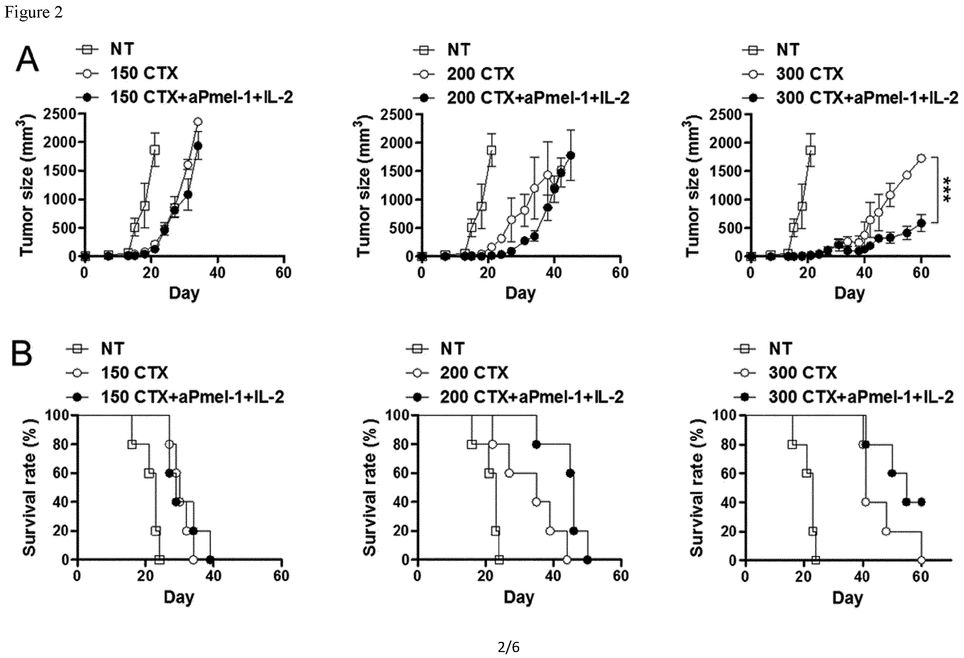

As a result, as shown in FIG. 2A and 2B, in mice (NT) in which only B16-F10 was implanted and PBS was administered, a cancer cell size exceeded 2000 mm³ within only 20 days, and most of the mice died. In the 150 mg/kg CTX-administered mouse, cancer tissue grew more slowly, and the mouse survived for up to 35 days. Even when aPmel-1 and IL-2 were additionally administered, the growth of cancer tissue is similar to when only CTX was administered, and the maximum survival date was only 40 days. The 200 mg/kg CTX-administered mouse showed slower growth of cancer tissue than the 150 mg/kg CTX-administered mouse, and survived for up to 45 days. When aPmel-1 and IL-2 were additionally administered, cancer tissue grew more slowly than when only CTX was administered, but had a similar size after 40 days and survived for up to 50 days. The 300 mg/kg CTX-administered mouse showed slower growth of cancer tissue than when 200 mg/kg CTX-administered mouse, and survived for up to 60 days. When aPmel-1 and IL-2 were additionally administered, it was confirmed that cancer tissue grew more slowly than when only CTX was administered, and even after 60 days had a size of only approximately 600 mm³, and the mouse has a survival rate of 40%.

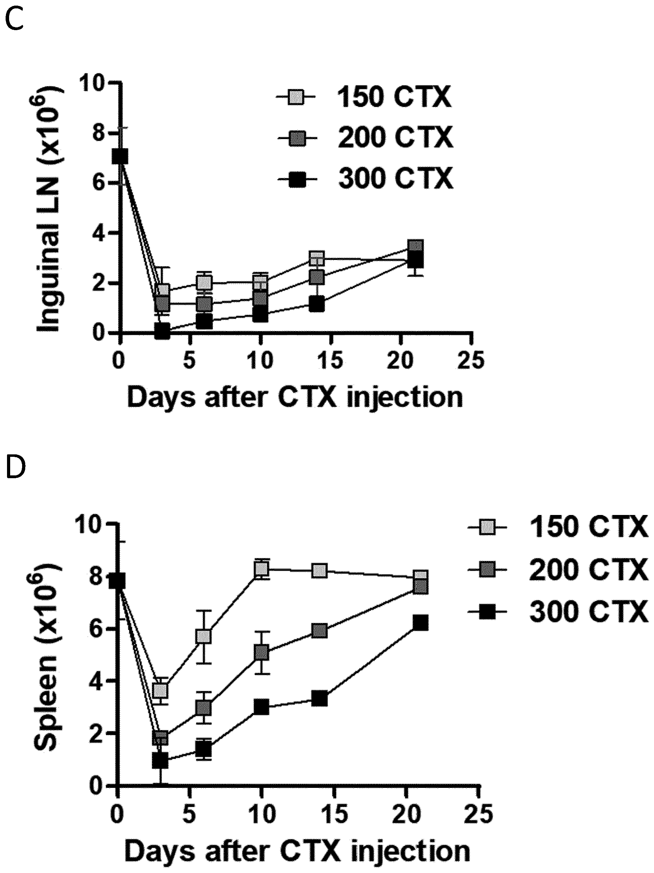

As the dose of the added CTX increases, the period of maintaining a transient immunodeficiency effect increases, confirming that the anticancer effect caused by aPmel-1 increases. However, when 300 mg/kg or more of CTX was administered, it can be toxic to the mouse, leading to the death of the mouse, and therefore it is not suitable for induction of transient immunodeficiency, and when 150 mg/kg CTX was administered, a transient immunodeficiency effect was insignificant. As shown in FIGS. 2C and 2D, even when 200 mg/kg CTX was administered, since it was confirmed that the immunodeficiency effect was maintained for approximately 2 weeks, the dose of the added CTX was 200 mg/kg. However, it was confirmed that this short period achieved a sufficient anticancer effect from aPmel-1.

### [Example 3]

### Effect of CD4 depletion on anticancer effect of Pmel-1 CD8 T cells

When partial immunodeficiency was continuously induced using a depleting anti-CD4 monoclonal antibody, it was intended to confirm whether it affects the anticancer effect of cancer antigen-specific CD8 T cells. It was also intended to confirm whether the number of Thy1.1⁺Pmel-1 CD8⁺ T cells and the inherent number of CD8 T cells of a cancer patient were changed.

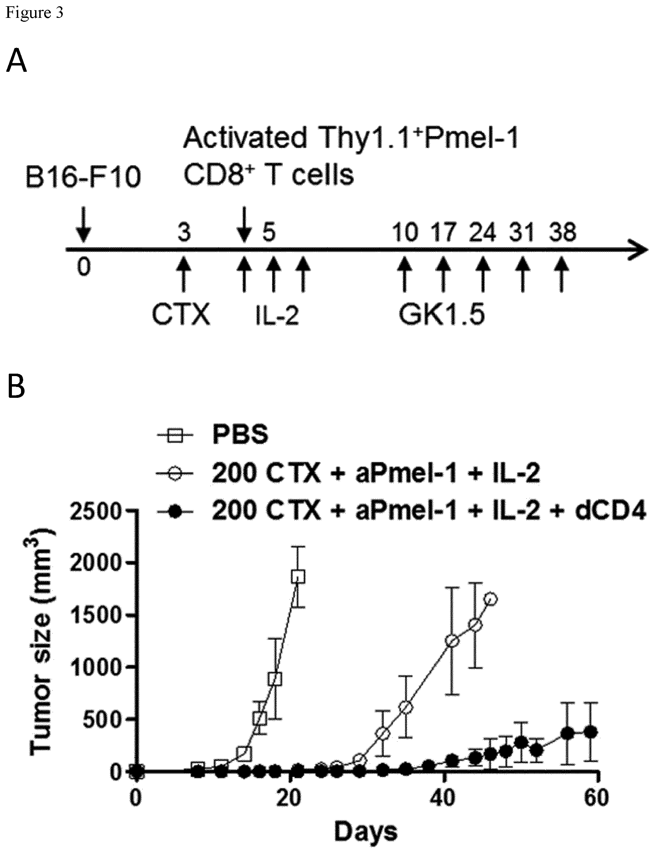

Specifically, experimental groups were prepared in the same manner as in Example 2. When continuous immunodeficiency was induced, from 10 days after B16-F10 implantation, 200 µg of GK1.5 (depleting anti-CD4 monoclonal antibody) was intraperitoneally administered five times at intervals of 8 days (FIG. 3A). To confirm the change in CD8 T cell number, at 10, 17, 24, 31 and 38 days after the cancer cell administration, blood was collected from mice of each experimental group, stained with anti-CD45, anti-CD8 and anti-Thy1.1 antibodies, and then a proportion of Thy1.1⁺CD8⁺ cells was analyzed from CD45-positive cells through flow cytometry. Particularly, at 20 days after the cancer cell administration, the proportion and number of CD8 T cells in each organ were analyzed.

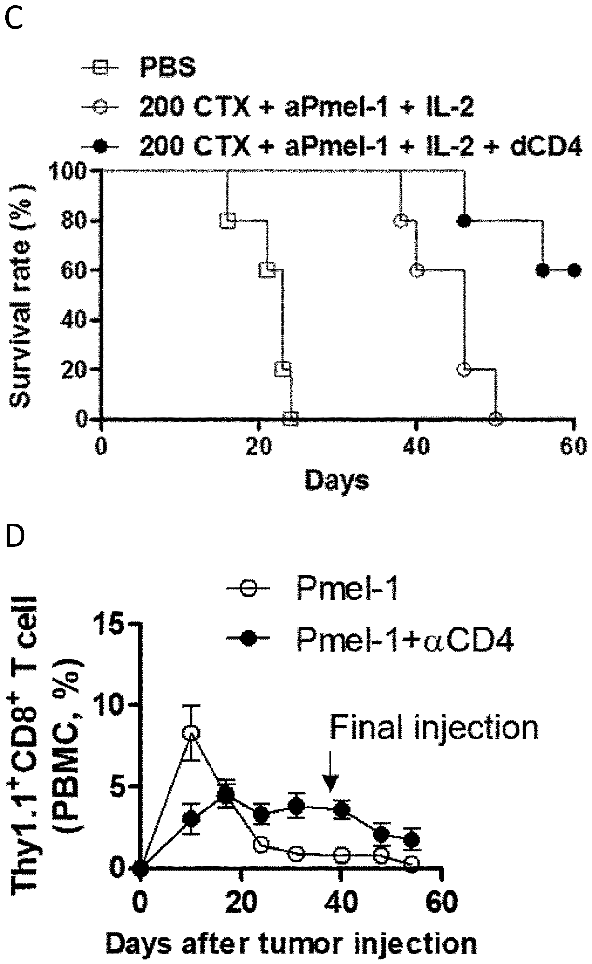

As a result, as shown in FIGS. 3B and 3C, mice (PBS) in which only B16-F10 was implanted and PBS was administered had a cancer cell size of more than 2000 mm³ within only 20 days, and most of the mice died. The mice to which aPmel-1 CD8 T cells and IL-2 were administered after 200 mg/kg CTX was administered began to show the rapid growth of cancer cells from 25 days, and survived for up to approximately 50 days. When the depleting anti-CD4 monoclonal antibody was administered under the same conditions as described above, the cancer cells grew insignificantly from day 35, but compared with that before the depleting anti-CD4 monoclonal antibody was administered, the growth of the cancer cells was slowed a maximum of 8-fold or more, and thus even after 60 days, the size was less than 500 mm³. In addition, 60% of the mice survived for 60 days or more. Even compared with the case in which 300 mg/kg CTX was administered and the depleting anti-CD4 monoclonal antibody was not administered (FIG. 2B), the size of a cancer cell was reduced 100 mm³ or more, and the proportion of the mice surviving for 60 days or more also increased 20% or more.

In addition, as shown in FIG. 3D, when the depleting anti-CD4 monoclonal antibody was continuously administered, compared to the case without administration, the proportion of the Thy1.1⁺CD8⁺ T cells was maintained at a higher level. Additionally, after the administration of the depleting anti-CD4 monoclonal antibody was stopped, the proportion of the Thy1.1⁺CD8⁺ T cells was gradually reduced after 4 to 5 days.

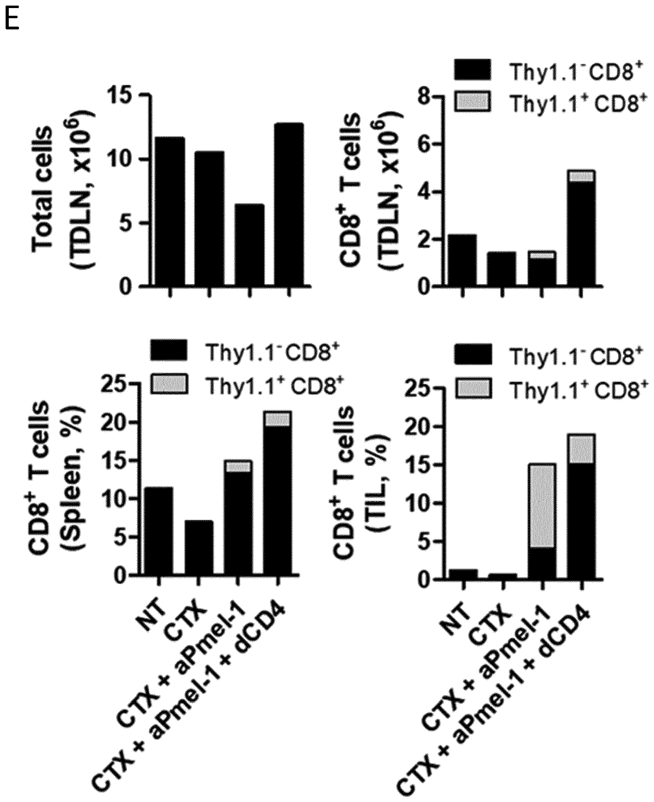

Particularly, as shown in FIG. 3E, compared with when CTX and aPmel-1 were administered, when a depleting anti-CD4 monoclonal antibody (dCD4) was additionally administered to the inguinal lymph node (TDLN), the total cell number increased. Among these, the number of CD8 T cells increased approximately 2.5 fold, compared with the case in which only CTX and aPmel-1 were administered, and particularly, it was confirmed that the CD8 T cells (Thy1.1⁻CD8⁺ T cells, black bar) in C57BL/6 mouse, compared with the administered Thy1.1⁺CD8⁺ T cells (gray bar), greatly increases. The proportion of CD8 T cells in the spleen also increased similar to the result of the inguinal lymph node.

In the case of CD8 T cells (TIL) invading cancer tissue, when only CTX and aPmel-1 were administered, the proportion of Thy1.1⁺CD8⁺ T cells was highly exhibited, but when a depleting anti-CD4 monoclonal antibody was additionally administered, the proportion of Thy1.1⁻CD8⁺ T cells was higher.

As a result, when partial immunodeficiency was continuously maintained through anti-CD4 monoclonal antibody depletion (anti-CD4 depletion), since *in vivo* proliferation of the administered CD8 T cells may be promoted, and the proliferation of CD8 T cells, which is inherent in a cancer patient, is induced, it is determined that the anticancer effect of the T cell therapy product, that is, aPmel-1, may increase.

### [Industrial Applicability]

The immunodeficiency-maintaining effect using a depleting anti-CD4 monoclonal antibody provided in the present invention can allow the effects of various anticancer therapies such as a T cell therapy product to be sufficiently exhibited in various cancer patients and thus can be effectively used in industries associated with prevention or treatment of cancer, indicating high industrial availability.

## Claims

1. A method of preventing or treating cancer, comprising:
i) inducing transient immunodeficiency in a cancer patient;
ii) administering cancer antigen-specific CD8 T cells and IL-2; and
iii) inducing continuous immunodeficiency.

2. The method of claim 1, wherein the transient immunodeficiency is induced by irradiation or the administration of an anticancer agent.

3. The method of claim 2, wherein the anticancer agent is one or more selected from the group consisting of cyclophosphamide and fludarabine.

4. The method of claim 1, wherein the cancer antigen is any one or more autologous cancer antigens selected from the group consisting of human telomere reverse transcriptase (hTERT), Wilm's tumor antigen 1 (WT-1), NY-ESO-1, melanoma-associated antigen (MAGE), carcinoembryonic antigen (CEA), CA-125, MUC-1 and melanoma antigen recognized by T cells 1 (MART-1).

5. The method of claim 1, wherein the continuous immunodeficiency is induced by a depleting anti-CD4 monoclonal antibody.

6. The method of claim 5, wherein the depleting anti-CD4 monoclonal antibody is administered twice or more at intervals of approximately 5 to 8 days.

7. The method of claim 1, wherein the cancer in Step i) is any one selected from the group consisting of lung cancer, stomach cancer, breast cancer, colon cancer, liver cancer, prostate cancer, uterine cancer, brain cancer and sarcomas.

8. A method of maintaining immunodeficiency, comprising:
i) inducing transient immunodeficiency in a cancer patient; and
ii) administering a depleting anti-CD4 monoclonal antibody to the cancer patient in which the transient immunodeficiency is induced.

9. The method of claim 8, wherein the cancer of Step i) is any one selected from the group consisting of lung cancer, stomach cancer, breast cancer, colon cancer, liver cancer, prostate cancer, uterine cancer, brain cancer and sarcomas.

10. The method of claim 8, wherein the transient immunodeficiency is induced by irradiation or the administration of an anticancer agent.

11. The method of claim 8, wherein the depleting anti-CD4 monoclonal antibody is administered twice or more at intervals of approximately 5 to 8 days.

12. A composition for maintaining immunodeficiency, comprising a depleting anti-CD4 monoclonal antibody.

13. The composition of claim 12, wherein the composition is administered twice or more at intervals of approximately 5 to 8 days.

14. The composition of claim 12, wherein when the composition is treated, a period of maintaining immunodeficiency is approximately 10 days or more after the treatment of the composition.

15. A composition for helping an anticancer T cell therapy product, comprising a depleting anti-CD4 monoclonal antibody.

16. The composition of claim 15, which is administered twice or more at intervals of approximately 5 to 8 days.

17. The composition of claim 15, wherein the cancer is any one selected from the group consisting of lung cancer, stomach cancer, breast cancer, colon cancer, liver cancer, prostate cancer, uterine cancer, brain cancer and sarcomas.

18. A pharmaceutical composition used in prevention or treatment of cancer, comprising:
a depleting anti-CD4 monoclonal antibody, cancer antigen-specific CD8 T cells, an immunodeficiency inducer and IL-2.

19. The pharmaceutical composition of claim 18, wherein the immunodeficiency inducer is one or more selected from the group consisting of cyclophosphamide and fludarabine.

20. The pharmaceutical composition of claim 18, wherein the cancer is any one selected from the group consisting of the lung cancer, stomach cancer, breast cancer, colon cancer, liver cancer, prostate cancer, uterine cancer, brain cancer and sarcomas.
